# EUROPEAN PATENT SPECIFICATION

(11) **EP 1 617 826 B1**
(45) Date of publication and mention of the grant of the patent: **25.06.2008**
(21) Application number: 04760192.7
(22) Date of filing: 19.04.2004
(51) Int. Cl.: A61K 9/50, A61K 31/663, A61K 31/734

(54) **THE DISPERSIBLE ALENDRONATE MICROPARTICLE FORMULATION**
DISPERGIERBARE ALENDRONAT-MIKROTEILCHENFORMULIERUNG
FORMULATION DISPERSIBLE DE MICROPARTICULES D'ALENDRONATE

(30) Priority: 18.04.2003 TR 200300510
(43) Date of publication of application: 25.01.2006
(73) Proprietor: Sanovel Ilaç Sanayi Ve Ticaret Anonim Sirketi, 34398 Istanbul (TR)
(72) Inventor: ÖNER, Levent, 06610 ANKARA (TR); IFTER, Ümit, C Blok Kat 2 Maslak, 34398 ISTANBUL (TR); SAKARYA, Nisa, C Blok Kat 2 Maslak, 34398 ISTANBUL (TR); TÜRKYILMAZ, Ali, C Blok Kat 2 Maslak, 34398 ISTANBUL (TR)
(74) Representative: Iskender, Ibrahim
(86) International application number: PCT/TR2004/000024
(87) International publication number: WO 2004/096189

(56) References cited:
- WO-A-01/66119
- WO-A-98/56360
- WO-A-03/043641
- US-A- 4 140 760
- US-A- 5 773 429
- US-A- 5 853 759
- US-A- 6 143 326

## Description

### TECHNICAL FIELD

This invention relates to the pharmaceutical dosage forms that are packaged into sachets, which contain therapeutic amounts of alendronate (alendronate sodium or alendronate monosodium trihydrate or pharmaceutically acceptable derivatives) micro-particles that are prevented to be released into saliva, and alginic acid or sodium alginate and at least one sweetener or a mixture of sweeteners, to be administered orally after dispersed in a glass of 250 ml; water.

The above-mentioned invention is associated with the decrease of potential side effects of alendronate that may arise in esophagus and stomach. The methods in this invention are:
- the use of sodium alginate or alginic acid to prevent oesophageal reflux, ulcer and heartburn that may arise during alendronate use
- prevention any irritation related to alendronate sodium during its passage through esophagus by ensuring the release of it in stomach.

### BACKGROUND OF THE INVENTION

Alendronate sodium is chemically described as (4-amino-1-hydroxybutylidene) bisphosphonic acid monosodium salt trihydrate. Patients complain about heartburn after use of alendronate. Alendronate sodium tablets are recommended to be used with a glass of water. In U.S. Patent No. 5.853.759, it has been disclosed that alendronate tablets taken without a glass of water may cause irritation.

Currently therapies with alendronate may be grouped as permanent treatments with daily doses and treatments to be applied with given intervals (once in three days, once in a week or once in 15 days). It is known that in both treatment forms alendronate causes disorders such as esophageal reflux, heartburn and esophagitis. The patent with PCT publication No. of WO 99/04773, relates to alendronate treatment with given intervals such as its use once a week, once in two weeks or once a month to decrease its gastrointestinal side effects. Additionally, the use of the pharmaceutical compound in combination with H2 receptor blockers and/or proton pump inhibitors is disclosed in this patent.

The effervescent alendronate formulations are disclosed in U.S. Patent No. of 5.853.759 and in addition to that, procedures for preparing the liquid alendronate formulations are disclosed in PCT publication No. of WO 98/14196. In these patents, alendronate is in dissolved form, i.e., alendronate that gets in contact with saliva is the dissolved alendronate.

In the patent with PCT publication No. of WO 01/01991, bisphosphonate tablets with improved surface characteristics have been disclosed.

The patent with PCT publication No. of WO 02/00204 relates to gastric passage of alendronic acid and its salts, and the effect of tannic acid and super dispersants.

The patent with PCT publication No. of WO 98/56360 relates to rapid passage of bisphosphonate tablets through esophagus.

In the patent with PCT publication No. of WO 97/39755, coated dosage forms that contain ibandronate in inner phase to ensure rapid release have been disclosed.

The patent with PCT publication No. of WO 95/00881 contains studies associated with enteric coated alendronate pharmaceutical dosage forms. One of the different aspects of this patent from that study is the use of polymers as coating material that are resistant to gastric acid and are opened at intestinal pH.

Sodium alginate is used in cases of gastrointestinal reflux, heartburn and esophagitis. In the Patent numbered EP-A-0059221, the protective effect of alginic acid and its water soluble salts in gastrointestinal channel have been disclosed. In the patent with PCT publication No. of WO 01/66119, the compositions which cover the sachet formulations of alginic acid and sodium alginate are disclosed and these do not cover the combination of alendronate microparticles with sodium alginate and alginic acid that are coated with polymers resistant to salivary pH. It has been cited that sodium alginate disclosed in the patent with No. of 99/04773, may be used as an ingredient in preparation of pharmaceutical dosage forms but the addition of sodium alginate in therapeutic amounts to the formulation to compensate the esophageal reflux and gastric side effects of alendronate has not been cited. Sodium alginate or alginic acid may be obtained commercially from FMC or Monsanto (for example; Protanal LFR 5/60 or Munucol LB).

### SUMMARY OF THE INVENTION

This invention relates to the pharmaceutical dosage forms that are packaged into sachets, which contain alendronate microparticles coated with a polymer resistant to salivary pH, and the therapeutic amount of alginic acid or sodium alginate and at least one sweetener or a mixture of sweeteners, to be administered orally after dispersed in a glass of 250 ml. water.

Side effects occur with alendronate depending on its irritation of esophagus. To prevent this irritation one of the approaches of this invention is to prevent the contact of alendronate particles with esophagus. To ensure this, alendronate particles are coated with a polymer resistant to salivary pH. Microparticles of alendronate may be prepared by extrusion-rolling, vessel or liquidized bed procedures. The prepared particles are coated with a polymer resistant to salivary pH in a vessel or liquidized bed. For the coating purposes polymers such as aminoalkylmethacrylate copolymers and polyvinyl acetate diethylaminoacetate polymers that are insoluble (neutral pH) in saliva, but soluble in gastric pH may be used. Eudragit E (polymethacrylates, polyvinyl acetate diethylaminoacetate and poly butyl methacrylate / 2-dimethylamino-ethyl methacrylate / methyl methacrylate copolymers) which that is commercially produced in Röhm Pharma can be used in the coating.

The prepared alendronate microparticles are mixed with sodium alginate in a dry environment. After adding the sweetener and the other excipients, the sachet is prepared

To provide a good taste to the formulation, aspartame, potassium acesulfame, sodium saccharine, sucrose and its derivatives, polyols such as mannitol and sorbitol and monoammonium glycyrrhizinate may be used alone or as a mixture.

In the manufacture of the sachets, diluents (i.e., lactose, microcrystalline cellulose) lubricants (i.e., magnesium stearate, talc and PEG 6000), disintegrants (i.e. carboxymethylcellulose, crospovidone, carboxymethyl starch), surfactants, flavors and aromas may be used alone or as mixtures.

To prevent irritant effect of alendronate in mouth and esophagus, alendronate particles are coated with polymers insoluble in neutral pH (neutral pH corresponds to salivary pH). This polymer should be soluble in the gastric pH (pH 1-4) but not in the salivary pH (pH 6-7.5).

The prepared dispersible microparticles sachet formulation when dispersed in a glass of 250ml. water at the degree of 25°C, alendronate should not be released from the coated alendronate particles preferably in 3 minutes. At the end of 3rd minute, the release of not more 10% w/v of alendronate is permissible.

When with the prepared dispersible microparticles sachet formulation, the dissolution assay is performed in 0.1 N HCl (gastric madium, pH 1.2) at 900 rpm (USP XXIV, paddle method) not less than 85% of alendronate should be dissolved at the end of the 30 minutes.

### EXAMPLE

### a. The manufacture of the alendronate microparticles that are resistant to salivary pH

Alendronate particle are aggregated with the following mixture:

| | |
|---|---|
| Alendronate | 13.05 mg - 91.35 mg |
| PVP K30 | 1 mg - 14 mg |
| Ethanol | 6 mg - 84 mg |

By the use of the spray coating procedure, alendronate is aggregated with polyvinylprrolidone dissolved in ethanol. The aggregated particles are coated with the following mixture.

| | |
|---|---|
| Eudragit E100 | 10 mg - 280 mg |
| Ethanol | 50 mg - 400 mg |
| Acetone | 50 mg - 400 mg |
| Colloidal silica | 2 mg - 15 mg |

Eudragit E100 means poly (butyl methacrylate, 82-dimethyl amino ethyl) methacrylate, methyl methacrylate) 1:2:1.

### b. Preparation of the sachet

An amount equivalent to 10 mg alendronate acid is taken from the prepared coated microparticles.

| | |
|---|---|
| Coated microparticles equivalent to 10 mg alendronic acid | 26 mg |
| Sucrose | 4648.9 mg |
| Sodium alginate (Protanal LFR 5/60) | 300 mg |
| Saccharine sodium | 0.1 mg |
| PEG 6000 (lubricant) | 25 mg |

### Dissolution Test

The dissolution test has been carried out in 900 ml of 0.1 N HCl (pH 1.2). It has been performed by the use of paddle method under the conditions given in USP XXIV at 50 rpm. At the end of 30 minutes, 89% of alendronate has been dissolved.

Dissolution test of alendronate from the mixture contained in the sachet in salivary pH (neutral pH, pH 6.5):

Since the prepared sachet mixture is to be used after it is dispersed in a glass of water; the mixture is dispersed in a glass of 250 ml. water at the degree of 25°C and 3 minutes after at pH 6.5 4% of alendronate has been dissolved.

## Claims

1. A dispersible pharmaceutical formulation for oral administration at therapeutic doses which comprises alendronate microparticles, and alginic acid or sodium alginate or admixtures thereof **characterized in that** said alendronate microparticles are coated with a polymer resistant to salivary pH of 6- 7.5, but soluble in gastric pH of 1-4.

2. A dispersible pharmaceutical formulation as claimed in claim 1, wherein said alendronate microparticles are preferably alendronate monosodium trihydrate or pharmaceutically acceptable derivatives.

3. A dispersible pharmaceutical formulation as claimed in claim 1, wherein said coating polymer is selected from polymethacrylates, polyvinyl acetate diethylaminoacetate and poly butyl methacrylate / dimethylamino-ethyl methacrylate / methyl methacrylate copolymers or mixtures thereof.

4. A dispersible pharmaceutical formulation as claimed in claim 3, wherein, said polymer is preferably Poly (butyl methacrylate, (2-dimethyl aminoethyl) methacrylate, methyl methacrylate) 1:2:1.

5. A formulation as claimed in claims 1 - 4, wherein the alendronate microparticles have the following formulation:
Material
| CORE | Range |
|---|---|
| | (%w/w) |
| Alendronate | % 1-90 |
| Additives to make | %0-80 |
| microparticles | |
| COATING | Range |
|---|---|
| | (%w/w) |
| Polymethacrilates | %1-80 |
| Ethanol | %0-80 |
| Acetone | %0-80 |
| Colloidal silica | %0-80 |

6. A formulation as claimed in claims 1 - 5, wherein the alendronate microparticles have the following formulation:
Material
| CORE | Range (%w/w) |
|---|---|
| Alendronate | %40-70 |
| Additives to make microparticles | %1-55 |
| (preferably ethanol or/ and PVP K30 etc) | |
| COATING | Range (%w/w) |
|---|---|
| Polymethacrylates (preferably Poly (butyl methacrylate, (2-dimethyl amino ethyl) methacrylate, methyl methacrylate) | %3-40 |
| 1:2:1. | |
| Ethanol | %20-50 |
| Acetone | %20-50 |
| Colloidal silica | %0, 01-25 |

7. A dispersible pharmaceutical formulation as claimed in any of the preceding claims, wherein said polymer provides that alendronate dissolves in 900 ml 0,1 N HCl at the rate of not less than 85% within 30 minutes at the range of pH 1-4.

8. A dispersible pharmaceutical formulation as claimed in any of the preceding claims, said polymer provides that the alendronate disperses in 3 minutes in the range between 0-10% w/v in 250ml. water at 25°C at pH 6- 7,5.

9. A dispersible pharmaceutical formulation as claimed in any of the preceding claims, wherein said formulation further comprises lubricants, diluents, flavors and sweeteners or mixtures thereof.

10. A dispersible pharmaceutical formulation as claimed in claim 9, where in the diluent is preferably selected from lactose and microcrystalline cellulose or admixtures thereof.

11. A dispersible pharmaceutical formulation as claimed in claim 9, wherein the sweetener is preferably selected from aspartame, potassium acesulfame, monoammonium glycyrhizinate, sodium saccharine, sucrose and derivatives thereof, polyols and derivatives thereof, being used alone or in combination thereof.

12. A method for the preparation of a dispersible pharmaceutical formulation for oral administration at therapeutic doses which process comprises;
- preparing a powder or granulate comprising of alendronate microparticles
- coating said alendronate microparticles with a polymer resistant to salivary pH of 6-7.5, but soluble in gastric pH of 1-4.
- compacting coated alendronate microparticles with alginic acid or sodium alginate or admixtures thereof to produce a dosage form.

13. A method as claimed in claim 12, wherein said coating polymer is selected from polymethacrylates, polyvinyl acetate diethylaminoacetate and poly butyl methacrylate / dimethylamino- ethyl methacrylate / methyl methacrylate copolymers or mixtures thereof.

14. A method as claimed in claim 13, wherein said polymer is preferably Poly (butyl methacrylate, (2-dimethyl aminoethyl) methacrylate, methyl methacrylate) 1:2:1.

15. A method as claimed in claims 12 - 14, wherein said formulation further comprises lubricants, diluents, flavors and sweeteners or mixtures thereof.

16. Use of an alendronate microparticle formulation, wherein alendronate microparticles are coated with a polymer resistant to salivary pH of 6- 7.5, but soluble in gastric pH of 1-4, in combination with alginic acid or sodium alginate or admixtures thereof for the manufacture of a medicament for inhibiting the side effects of alendronate.

17. Use of an alendronate microparticle formulation as claimed in claim 16, wherein the said side effects of alendronate comprise esophageal reflux, ulcer, heartburn and esophagitis.

## Patentansprüche

1. Dispergierbare pharmazeutische Formulierung für die orale Verabreichung in therapeutischen Dosen, welche Alendronat-Mikropartikel und Alginsäure oder Natriumalginat oder Beimischungen davon aufweist, **dadurch gekennzeichnet, dass** Alendronat-Mikropartikel mit einem Polymer beschichtet sind, das gegenüber einem pH-Wert von 6-7,5 im Speichel resistent, bei einem gastrischen pH-Wert von 1-4 aber löslich ist.

2. Dispergierbare pharmazeutische Formulierung nach Anspruch 1, wobei es sich bei den Alendronat-Mikropartikeln vorzugsweise um Alendronatmononatriumtrihydrat oder pharmazeutisch annehmbare Derivate handelt.

3. Dispergierbare pharmazeutische Formulierung nach Anspruch 1, wobei das Beschichtungspolymer aus Polymethacrylaten, Polyvinylacetatdiethylaminoacetat und Polybutylmethacrylat/Dimethylaminoethylmethacrylat/Methylmethacrylat-Copolymeren oder Gemischen davon ausgewählt ist.

4. Dispergierbare pharmazeutische Formulierung nach Anspruch 3, wobei es sich bei dem Polymer vorzugsweise um Poly(butylmethacrylat,(2-dimethylaminoethyl)methacrylat, methylmethacrylat) 1:2:1 handelt.

5. Formulierung nach Anspruch 1-4, wobei die Alendronat-Mikropartikel folgende Formulierung aufweisen:
Material
| KERN | Bereich (% Gew./Gew.) |
|---|---|
| Alendronat | % 1-90 |
| Additive zur Herstellung von Mikropartikeln | % 0-80 |
| BESCHICHTUNG | Bereich |
|---|---|
| | (% Gew./Gew.) |
| Polymethacrylate | % 1-80 |
| Ethanol | % 0-80 |
| Aceton | % 0-80 |
| Kolloid-Kieselerde | % 0-80 |

6. Formulierung nach Anspruch 1-5, wobei die Alendronat-Mikropartikel folgende Formulierung aufweisen:
Material
| KERN | Bereich (% Gew./Gew.) |
|---|---|
| Alendronat | % 40-70 |
| Additive zur Herstellung von Mikropartikeln (vorzugsweise Ethanol und/oder PVP K30 etc.) | % 1-55 |
| BESCHICHTUNG | Bereich (% Gew./Gew.) |
|---|---|
| Polymethacrylate (vorzugsweise Poly(butylmethacrylat, (2-dimethylaminoethyl)methacrylat, methylmethacrylat) 1:2:1 | % 3-40 |
| Ethanol | % 20-50 |
| Aceton | % 20-50 |
| Kolloid-Kieselerde | % 0,01-25 |

7. Dispergierbare pharmazeutische Formulierung nach einem der vorherigen Ansprüche, wobei das Polymer dafür sorgt, dass sich Alendronat in 900 ml 0,1 N HCl bei der Rate von mindestens 85% innerhalb von 30 Minuten im pH-Bereich von 1-4 auflöst.

8. Dispergierbare pharmazeutische Formulierung nach einem der vorherigen Ansprüche, wobei das Polymer dafür sorgt, dass sich Alendronat in 250 ml Wasser bei 25°C bei pH 6-7,5 in 3 Minuten im Bereich zwischen 0-10 % Gew./Vol. auflöst.

9. Dispergierbare pharmazeutische Formulierung nach einem der vorherigen Ansprüche, wobei die Formulierung des Weiteren Gleitmittel, Verdünnungsmittel, Aromastoffe und Süßungsmittel oder Gemische davon aufweist.

10. Dispergierbare pharmazeutische Formulierung nach Anspruch 9, wobei das Verdünnungsmittel vorzugsweise aus Laktose und mikrokristalliner Cellulose oder Beimischungen davon ausgewählt ist.

11. Dispergierbare pharmazeutische Formulierung nach Anspruch 9, wobei das Süßungsmittel vorzugsweise aus Aspartam, Kaliumacesulfam, Monoammoniumglycyrhizinat, Natriumsaccharin, Saccharose und Derivaten davon, Polyolen und Derivaten davon ausgewählt ist, die alleine oder in einer Kombination davon verwendet werden.

12. Verfahren für die Herstellung einer Dispergierbaren pharmazeutische Formulierung für die orale Verabreichung in therapeutischen Dosen, wobei das Verfahren umfasst:
- Herstellen eines Pulvers oder Granulats, das Alendronat-Mikropartikel aufweist;
- Beschichten der Alendronat-Mikropartikel mit einem Polymer, das gegenüber einem pH-Wert von 6-7,5 im Speichel resistent, bei einem gastrischen pH-Wert von 1-4 aber löslich ist;
- Verdichten der beschichteten Alendronat-Mikropartikel mit Alginsäure oder Natriumalginat oder Beimischungen davon zur Herstellung einer Dosierungsform.

13. Verfahren nach Anspruch 12, wobei das Beschichtungspolymer aus Polymethacrylaten, Polyvinylacetatdiethylaminoacetat und Polybutylmethacrylat/Dimethylaminoethylmethacrylat/Methylmethacrylat-Copolymeren oder Gemischen davon ausgewählt ist.

14. Verfahren nach Anspruch 13, wobei es sich bei dem Polymer vorzugsweise um Poly(butylmethacrylat,(2-dimethylaminoethyl)methacrylat, methylmethacrylat) 1:2:1 handelt.

15. Verfahren nach Anspruch 12-14, wobei die Formulierung des Weiteren Gleitmittel, Verdünnungsmittel, Aromastoffe und Süßungsmittel oder Gemische davon aufweist.

16. Verwendung einer Alendronat-Mikropartikelformulierung, wobei die Alendronat-Mikropartikel mit einem Polymer beschichtet sind, das gegenüber einem pH-Wert von 6-7,5 im Speichel resistent, bei einem gastrischen pH-Wert von 1-4 aber löslich ist, in Kombination mit Alginsäure oder Natriumalginat oder Beimischungen davon zum Herstellen eines Medikaments zum Hemmen der Nebenwirkungen von Alendronat.

17. Verwendung einer Alendronat-Mikropartikelformulierung nach Anspruch 16, wobei die Nebenwirkungen von Alendronat ösophagealen Reflux, Ulkus, Sodbrennen und Ösophagitis umfassen.

## Revendications

1. Formulation pharmaceutique dispersible destinée à une administration par voie orale à des doses thérapeutiques, qui comprend des microparticules d'alendronate, et de l'acide alginique ou de l'alginate de sodium ou des mélanges de ceux-ci, **caractérisée en ce que** lesdites microparticules d'alendronate sont enrobées d'un polymère résistant à un pH salivaire de 6 à 7,5, mais soluble à un pH gastrique de 1 à 4.

2. Formulation pharmaceutique dispersible selon la revendication 1, dans laquelle lesdites microparticules d'alendronate sont de préférence l'alendronate monosodique trihydraté ou des dérivés pharmaceutiquement acceptables.

3. Formulation pharmaceutique dispersible selon la revendication 1, dans laquelle ledit polymère d'enrobage est choisi parmi les polyméthacrylates, le polyvinyl acétate diéthylaminoacétate et les copolymères de poly butyl méthacrylate/diméthylamino-éthyl méthacrylate/méthyl méthacrylate ou des mélanges de ceux-ci.

4. Formulation pharmaceutique dispersible selon la revendication 3, dans laquelle, ledit polymère est de préférence le poly (butyl méthacrylate, (2-diméthyl aminoéthyl) méthacrylate, méthyl méthacrylate) 1/2/1.

5. Formulation selon les revendications 1 à 4, dans laquelle les microparticules d'alendronate ont la formulation suivante :
Matériau
| COEUR | Plage |
|---|---|
| | (% pds/pds) |
| Alendronate | % 1-90 |
| Additifs pour la fabrication des microparticules | % 0-80 |
| ENROBAGE | Plage (% pds/pds) |
|---|---|
| Polyméthacrylates | % 1-80 |
| Ethanol | % 0-80 |
| Acétone | % 0-80 |
| Silice colloïdale | % 0-80 |

6. Formulation selon les revendications 1 à 5, dans laquelle les microparticules d'alendronate ont la formulation suivante :
Matériau
| COEUR | Plage (% pds/pds) |
|---|---|
| Alendronate | % 40-70 |
| Additifs pour la | % 1-55 |
| fabrication des microparticules | |
| (de préférence éthanol | |
| et/ou PVP K30 etc) | |
| ENROBAGE | Plage |
|---|---|
| | (% pds/pds) |
| Polyméthacrylates (de préférence poly (butyl méthacrylate, (2-diméthyl amino éthyl) méthacrylate, méthyl méthacrylate) 1/2/1. | %3-40. |
| Ethanol | % 20-50 |
| Acétone | % 20-50 |
| Silice colloïdale | % 0,01-25 |

7. Formulation pharmaceutique dispersible selon l'une quelconque des revendications précédentes, dans laquelle ledit polymère permet la dissolution de l'alendronate dans 900 mL de HC1 0,1 N à un taux pas inférieur à 85 % en 30 minutes à un pH dans la plage de 1 à 4.

8. Formulation pharmaceutique dispersible selon l'une quelconque des revendications précédentes, dans laquelle ledit polymère permet la dispersion de l'alendronate en 3 minutes dans la plage de 0 à 10 % pds/v dans 250 mL d'eau à 25°C à pH de 6 à 7,5.

9. Formulation pharmaceutique dispersible selon l'une quelconque des revendications précédentes, dans laquelle ladite formulation comprend en outre des lubrifiants, des diluants, des aromatisants et des édulcorants ou des mélanges de ceux-ci.

10. Formulation pharmaceutique dispersible selon la revendication 9, dans laquelle le diluant est de préférence choisi parmi le lactose et la cellulose microcristalline ou des mélanges de ceux-ci.

11. Formulation pharmaceutique dispersible selon la revendication 9, dans laquelle l'édulcorant est de préférence choisi parmi l'aspartame, l'acésulfame de potassium, le monoammonium glycyrrhizinate, la saccharine sodique, le sucrose et ses dérivés, les polyols et leurs dérivés, utilisés seuls ou en combinaison.

12. Procédé de préparation d'une formulation pharmaceutique dispersible destinée à une administration orale à des doses thérapeutiques, lequel procédé comprend :
- la préparation d'une poudre ou d'un granulat comprenant des microparticules d'alendronate
- l'enrobage desdites microparticules d'alendronate avec un polymère résistant à un pH salivaire de 6 à 7,5, mais soluble à un pH gastrique de 1 à 4
- le compactage des microparticules d'alendronate enrobées avec de l'acide alginique ou de l'alginate de sodium ou des mélanges de ceux-ci pour produire une forme posologique.

13. Procédé selon la revendication 12, dans lequel ledit polymère d'enrobage est choisi parmi les polyméthacrylates, le polyvinyl acétate diéthylaminoacétate et les copolymères de poly butyl méthacrylate/diméthylamino-éthyl méthacrylate/méthyl méthacrylate ou des mélanges de ceux-ci.

14. Procédé selon la revendication 13, dans lequel ledit polymère est de préférence le poly (butyl méthacrylate, (2-diméthyl aminoéthyl) méthacrylate, méthyl méthacrylate) 1/2/1.

15. Procédé selon l'une quelconque des revendications 12 à 14, dans lequel ladite formulation comprend en outre des lubrifiants, des diluants, des aromatisants et des édulcorants ou des mélanges de ceux-ci.

16. Utilisation d'une formulation de microparticules d'alendronate, dans laquelle les microparticules d'alendronate sont enrobées d'un polymère résistant à un pH salivaire de 6 à 7,5, mais soluble à un pH gastrique de 1 à 4, en combinaison avec de l'acide alginique ou de l'alginate de sodium ou des mélanges de ceux-ci pour la fabrication d'un médicament destiné à inhiber les effets secondaires de l'alendronate.

17. Utilisation d'une formulation de microparticules d'alendronate selon la revendication 16, dans laquelle lesdits effets secondaires de l'alendronate comprennent un reflux oesophagien, un ulcère, un pyrosis et une oesophagite.
